# EUROPÄISCHE PATENTSCHRIFT

(11) **EP 0 583 695 B1**
(45) Veröffentlichungstag und Bekanntmachung des Hinweises auf die Patenterteilung: **15.01.1997**
(21) Anmeldenummer: 93112551.2
(22) Anmeldetag: 05.08.1993
(51) Int. Cl.: C07C 51/10, C07C 51/02, C07C 53/02, C07C 53/04, C07C 53/06

(54) **Verfahren zur Herstellung von Ameisensäure aus Kohlenmonoxid und Wasser**
Process for the preparation of formic acid from carbon monoxide and water
Procédé pour la préparation de l'acide formique à partir de l'oxide de carbone et de l'eau

(30) Priorität: 19.08.1992 DE 4227394
(43) Veröffentlichungstag der Anmeldung: 23.02.1994
(73) Patentinhaber: BASF Aktiengesellschaft, 67063 Ludwigshafen (DE)
(72) Erfinder: Lippert, Ferdinand, Dr., D-6702 Bad Duerkheim (DE); Hoehn, Arthur, Dr., D-6700 Ludwigshafen (DE)

(56) Entgegenhaltungen:
- EP-A- 0 126 524
- DE-A- 3 903 664
- DE-C- 414 257
- FR-A- 421 227
- FR-A- 1 428 402

## Beschreibung

Die vorliegende Erfindung betrifft ein Verfahren zur Herstellung von Ameisensäure aus Kohlenmonoxid und Wasser in Gegenwart tertiärer Amine.

Aus der EP-A 248 259 ist die Herstellung von Ameisensäure aus Kohlenmonoxid und Wasser in einem Zweistufenprozeß bekannt, wobei in der ersten Stufe ein primäres oder sekundäres Hydroxyalkylamin mit Kohlenmonoxid carbonyliert und in der zweiten Stufe das so gebildete Hydroxyalkylformamid in Gegenwart eines sauren Katalysators zu Ameisensäure und Amin hydrolysiert wird. Aus dem Hydrolysegemisch entfernt man die Ameisensäure durch Strippen mit Wasserdampf unter Druck. Anschließend wird die erhaltene wäßrige Ameisensäure durch azeotrope Destillation mit Diisopropylether oder einem Ameisensäureester als Schleppmittel getrocknet. Unbefriedigend ist dabei die Hydrolyse des Formamids in der zweiten Stufe. Man erreicht zum einen nur einen maximalen Hydrolysegrad von 45 % (siehe Tabelle 2, Seite 10), wobei die Hydrolysetemperaturen zwischen 70 und 150°C liegen, und zum anderen erfolgt die Hydrolyse nur sehr langsam. Die Verweilzeiten liegen zwischen 4 und 20 Stunden.

In der DE-A-39 03 664 wird die Synthese von Ameisensäure aus Kohlenmonoxid und Wasser in Gegenwart von tertiären Hydroxyalkylaminen beschrieben. Aus Kohlenmonoxid und den tertiären Hydroxyalkylaminen bilden sich intermediär die entsprechenden Ameisensäureester. Die Darstellung der Ameisensäureester erfolgt zwar unter relativ milden Bedingungen, jedoch wird nur ein maximaler Umsatz von 22 % erreicht. Die anschließende Hydrolyse mit äquimolaren Mengen Wasser verläuft quantitativ. Es werden jedoch nur geringe Raum-Zeit-Ausbeuten erzielt (vgl. Beispiel 1: RZA = 22,1 g HCOOH/l/h; Beispiel 2: RZA = 21 g HCOOH/l/h). Die wäßrige Ameisensäurelosung wird wie in EP-A-248 259 beschrieben aufgearbeitet.

Der vorliegenden Erfindung lag daher die Aufgabe zugrunde, den zuvor genannten Nachteilen abzuhelfen.

Demgemäß wurde ein neues und verbessertes Verfahren zur Herstellung von Ameisensäure durch Umsetzung von Kohlenmonoxid mit Wasser in Gegenwart von Aminen bei Temperaturen von 100 bis 250°C und absoluten Drücken von 100 bis 350 bar zu Ammoniumformiaten und thermische Trennung solcher Ammoniumformiate in Ameisensäure und Amin gefunden, welches dadurch gekennzeichnet ist, daß man tertiäre Amine der allgemeinen Formel I 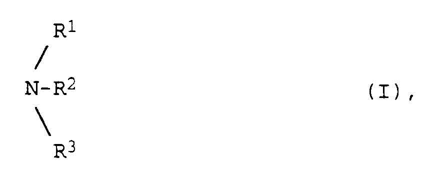 in der
- R¹,R²,R³: C₁- bis C₁₄-Alkyl, C₃- bis C₈-Cycloalkyl, Aryl und C₇- bis C₁₆-Aralkyl oder gemeinsam eine gegebenenfalls durch ein- bis vierfach C₁- bis C₄-Alkyl substituierte 1,4- oder 1,5-Alkylengruppe bedeutet, mit der Maßgabe, daß die Summe der Kohlenstoffatome von R¹, R² und R³ 3 bis 40 beträgt, N-Hexylmorpholin oder N,N'-Dimorpholinoethan
der Reaktionsmischung zusetzt.

Das erfindungsgemäße Verfahren kann wie folgt durchgeführt werden:

Wasser und das tertiäre Amin I, bevorzugt mit aliphatischen, araliphatischen und/oder aromatischen Resten, können in der Flüssigphase vorgelegt werden und Kohlenmonoxid anschließend aufgepreßt werden. Die Umsetzung von Kohlenmonoxid und Wasser in Gegenwart von tertiären Aminen kann man diskontinuierlich oder bevorzugt kontinuierlich bei Temperaturen von 100 bis 250°C, bevorzugt 150 bis 200°C und absoluten Drücken von 100 bar bis 350 bar, bevorzugt 200 bis 300 bar durchführen.

Kohlenmonoxid kann in reiner Form oder als Synthesegas in die Reaktion eingesetzt werden. Das Molverhältnis von Wasser zum Amin kann in weiten Grenzen variiert werden, geeignet sind Molverhältnisse von 0,2 : 1 bis 100 : 1, bevorzugt 20 : 1 bis 1 : 1, besonders bevorzugt 10 : 1 bis 2 : 1.

Als Reaktoren eignen sich Kessel, Blasensäulen oder Rieseltürme. Die Isolierung der Ameisensäure aus dem Reaktionsgemisch kann nach bekannten Trennoperationen wie Destillation oder Strippen erfolgen. Die thermische Trennung solcher Ammoniumformiate in Ameisensäure und freies Amin ist z.B. aus EP-A-1432 bekannt.

Die Substituenten R¹, R² und R³ in Verbindungen I haben folgende Bedeutungen:
R¹,R²,R³
- C₁- bis C₁₄-Alkyl wie Methyl, Ethyl, n-Propyl, iso-Propyl, n-Butyl, iso-Butyl, sec.-Butyl, tert.-Butyl, n-Pentyl, iso-Pentyl, sec.-Pentyl, neo-Pentyl, 1,2-Dimethylpropyl, n-Hexyl, iso-Hexyl, sec.-Hexyl, n-Heptyl, iso-Heptyl, n-Octyl, iso-Octyl, n-Nonyl, iso-Nonyl, n-Decyl, iso-Decyl, n-Undecyl, iso-Undecyl, n-Dodecyl und iso-Dodecyl, bevorzugt C₁- bis C₈-Alkyl wie Methyl, Ethyl, n-Propyl, iso-Propyl, n-Butyl, iso-Butyl, sec.-Butyl, tert.-Butyl, n-Pentyl, iso-Pentyl, sec.-Pentyl, neo-Pentyl, 1,2-Dimethylpropyl, n-Hexyl, iso-Hexyl, sec.-Hexyl, n-Heptyl, iso-Heptyl, n-Octyl, iso-Octyl, besonders bevorzugt C₁- bis C₄-Alkyl wie Methyl, Ethyl, n-Propyl, iso-Propyl, n-Butyl, iso-Butyl, sec.-Butyl und tert.-Butyl,
- C₃- bis C₈-Cycloalkyl wie Cyclopropyl, Cyclobutyl, Cyclopentyl, Cyclohexyl, Cycloheptyl und Cyclooctyl, bevorzugt Cyclopentyl, Cyclohexyl und Cyclooctyl,
- Aryl wie Phenyl, 1-Naphthyl, 2-Naphthyl, 1-Anthryl, 2-Anthryl und 9-Anthryl, bevorzugt Phenyl, 1-Naphthyl und 2-Naphthyl, besonders bevorzugt Phenyl,
- C₇- bis C₁₆-Aralkyl, bevorzugt C₇- bis C₁₂-Phenylalkyl wie Benzyl, 1-Phenethyl, 2-Phenethyl, 1-Phenyl-propyl, 2-Phenyl-propyl, 3-Phenyl-propyl, 1-Phenyl-butyl, 2-Phenyl-butyl, 3-Phenyl-butyl und 4-Phenyl-butyl, besonders bevorzugt Benzyl, 1-Phenethyl und 2-Phenethyl,
oder R¹ und R² oder R¹ und R³ oder R² und R³ gemeinsam
- eine gegebenenfalls durch ein- bis vierfach C₁- bis C₄-Alkyl substituierte 1,4- oder 1,5-Alkylengruppe mit der Maßgabe, daß die Summe der Kohlenstoffatome von R¹, R² und R³ in den Verbindungen I und III 3 bis 40 beträgt, wie -(CH₂)₄-, -(CH₂)₅-, -CH(CH₃)-CH₂-CH₂-, -CH₂-CH(CH₃)-CH₂-, bevorzugt 4 bis 24, besonders bevorzugt 6 bis 18.

Als tert.-Alkylamine kommen insbesondere N-Tri-ethyl-, N-Tributyl-, N-Tri-pentyl-, N-Tri-hexyl-, N-Tri-heptyl-, N-Tri-octylamin, Dimethyl-tetradecylamin, Diethyl- tetrahecyl-, Dimethyl-octyl-, Diethyl-octylamin oder auch N,N'-Dimorpholinoethan oder N-Hexyl-morpholin in Frage.

### Beispiele

### Beispiel 1

In einem 0,3-l-Autoklaven aus Hastelloy HC-4 mit Magnetrührer wurden 50,6g (0,5 mol) Triethylamin und 90g (5 mol) Wasser vorgelegt. Der Autoklav wurde verschlossen und Kohlenmonoxid wurde bis zu einem Druck von 50 bar aufgepresst. Anschließend wurde auf 200°C aufgeheizt und mit Kohlenmonoxid der gewünschte Gesamtdruck von 300 bar eingestellt. Nach einer Stunde wurde der Autoklav abgekühlt und der flüssige Austrag analysiert. Es wurden 12,4g (0,27 mol) Ameisensäure als Formiat, was einem Umsatz von 54 % bezogen auf eingesetztes Amin entspricht, gefunden. Die Raum-Zeit-Ausbeute betrug 41 g Ameisensäure/l/h.

### Beispiel 2

In analoger Weise wurden 50,6g (0,5 mol) Triethylamin und 90g (5 mol) Wasser 5 Stunden bei 200°C und 200 bar Gesamtdruck umgesetzt. Der Austrag enthielt 20,7g (0,45 mol) Ameisensäure als Formiat, was einem Umsatz von 90 % bezogen auf eingesetztes Amin und einer Raum-Zeit-Ausbeute von 14 g Ameisensäure/l/h entspricht.

### Beispiel 3

Analog Beispiel 2, nur bei einem Gesamtdruck von 250 bar. Es wurden 21,4g (0,46 mol) Ameisensäure als Formiat, was einem Umsatz von 93 % bezogen auf eingesetztes Triethylamin entspricht, gebildet.

### Beispiel 4

Es wurden 81g (0,8 mol) Triethylamin und 72g (4 mol) Wasser in einem 0,3-l-HC-Autoklaven vorgelegt und bei 200°C und 300 bar gerührt. In ca. 2 Stunden wurden 12,8g (0,28 mol) Ameisensäure als Formiat erzeugt, was einem Umsatz von 35 % bezogen auf Triethylamin und einer Raum-Zeit-Ausbeute von 21,3g Ameisensäure/l/h entspricht.

## Patentansprüche

1. Verfahren zur Herstellung von Ameisensäure durch Umsetzung von Kohlenmonoxid mit Wasser in Gegenwart von Aminen bei Temperaturen von 100 bis 250°C und absoluten Drücken von 100 bis 350 bar zu Ammoniumformiaten und thermische Trennung solcher Ammoniumformiate in Ameisensäure und Amin, dadurch gekennzeichnet, daß man tertiäre Amine der allgemeinen Formel I 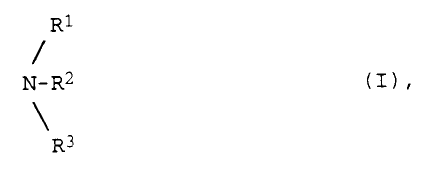 in der
R¹,R²,R³ C₁- bis C₁₄-Alkyl, C₃- bis C₈-Cycloalkyl, Aryl und C₇- bis C₁₆-Aralkyl oder gemeinsam eine gegebenenfalls durch ein- bis vierfach C₁- bis C₄-Alkyl substituierte 1,4- oder 1,5-Alkylengruppe bedeutet, mit der Maßgabe, daß die Summe der Kohlenstoffatome von R¹, R² und R³ 3 bis 40 beträgt, N-Hexylmorpholin oder N,N'-Dimorpholinoethan
der Reaktionsmischung zusetzt.

2. Verfahren zur Herstellung von Ameisensäure durch Umsetzung von Kohlenmonoxid mit Wasser nach Anspruch 1, dadurch gekennzeichnet, daß die Summe der Kohlenstoffatome von R¹, R² und R³ in den quartären Ammoniumformiaten von I 4 bis 20 beträgt.

3. Verfahren zur Herstellung von Ameisensäure durch Umsetzung von Kohlenmonoxid mit Wasser nach Anspruch 1, dadurch gekennzeichnet, daß man als tertiäre Amine I Triethylamin, Trihexylamin, Triheptylamin, Trioctylamin oder Dimethyltetradecylamin einsetzt.

## Claims

1. A process for preparing formic acid by reaction of carbon monoxide with water in the presence of amines at from 100 to 250°C and from 100 to 350 bar to give ammonium formates, and thermal cleavage of such ammonium formates into formic acid and amine, which comprises adding amines of the general formula I 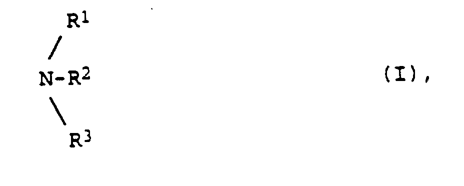 where
R¹, R² and R³ are C₁- to C₁₄-alkyl, C₃- to C₈-cycloalkyl, aryl or C₇- to C₁₆-aralkyl or together are a 1,4- or 1,5-alkylene group which is unsubstituted or mono- to tetrasubstituted by C₁- to C₄-alkyl, with the proviso that the sum of the carbon atoms of R¹, R² and R³ is from 3 to 40,
N-hexylmorpholine or N,N'-dimorpholinoethane to the reaction mixture.

2. A process for preparing formic acid by reaction of carbon monoxide with water as claimed in claim 1, wherein the sum of the carbon atoms of R¹, R² and R³ in the quaternary ammonium formates of I is from 4 to 20.

3. A process for preparing formic acid by reaction of carbon monoxide with water as claimed in claim 1, wherein the tertiary amine I employed is triethylamine, trihexylamine, triheptylamine, trioctylamine or dimethyltetradecylamine.

## Revendications

1. Procédé de préparation de l'acide formique par la conversion du monoxyde de carbone avec l'eau, en présence d'amines à des températures de 100 à 250°C et sous des pressions absolues de 100 à 350 bars, en formiates d'ammonium et scission thermique de ces formiates d'ammonium en acide formique et amine, caractérisé en ce que l'on ajoute au mélange réactionnel des amines de la formule générale (I) 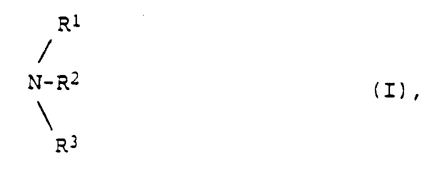 dans laquelle
R¹, R², R³ représentent chacun un radical alkyle en C₁-C₁₄, cycloalkyle en C₃-C₈, aryle et aralkyle en C₇ à C₁₆, ou représentent ensemble un radical 1,4- ou 1,5-alkylène, éventuellement substitué par de 1 à 4 radicaux alkyle en C₁-C₄, avec la condition que la somme des atomes de carbone de R¹, R² et R³, varie de 3 à 40, N-hexylmorpholine ou N, N'-dimorphilinoéthane.

2. Procédé de préparation de l'acide formique par la conversion du monoxyde de carbone et l'eau selon la revendication 1, caractérisé en ce que la somme des atomes de carbone de R¹, R² et R³, dans les formiates d'ammonium quaternaire de I varient de 4 à 20.

3. Procédé de préparation de l'acide formique par la réaction du monoxyde de carbone et l'eau selon la revendication 1, caractérisé en ce que l'on utilise à titre d'amines tertiaires I, la triéthylamine, la trihexylamine, la triheptylamine, la trioctylamine ou la diméthyltétradécylamine.
